# EUROPEAN PATENT APPLICATION

(11) **EP 0 604 667 A1**
(43) Date of publication of application: **06.07.1994**
(21) Application number: 93916204.6
(22) Date of filing: 22.07.1993
(51) Int. Cl.: C07C 229/46, A61K 31/215, C07C 237/24

(54) **TRANEXAMIC ACID DERIVATIVE AND DERMATOLOGIC PREPARATION CONTAINING THE SAME**

(30) Priority: 22.07.1992 JP 216290/92; 27.08.1992 JP 252322/92; 27.08.1992 JP 252323/92
(71) Applicant: SHISEIDO COMPANY LIMITED, Chuo-ku Tokyo 104 (JP)
(72) Inventor: OHNUMA, Manami Shiseido Research Center, Yokohama-shi Kanagawa 223 (JP); SUETSUGU, Masaru Shiseido Research Center, Yokohama-shi Kanagawa 223 (JP); SHINOJIMA, Satoshi Shiseido Research Center, Yokohama-shi Kanagawa 223 (JP); FUJINUMA, Yoshimori Shiseido Research Center, Yokohama-shi Kanagawa 223 (JP); MORIKAWA, Yoshihiro Shiseido Research Center, Yokohama-shi Kanagawa 223 (JP); YAMASE, Yuki Shiseido Research Center, Yokohama-shi Kanagawa 223 (JP); AKIYAMA, Naoe Shiseido Research Center, Yokohama-shi Kanagawa 223 (JP); KITAMURA, Kenji Shiseido Research Center, Yokohama-shi Kanagawa 223 (JP)
(74) Representative: Perry, Robert Edward
(86) International application number: JP9301021
(87) International publication number: WO9402444

(57) **Abstract**

A tranexamic acid derivative represented by general formula (I) and a salt thereof, wherein R represents a residue of a 6-membered ring containing an ester or amide linkage. It has excellent effects of beautifying and ameliorating chaps.

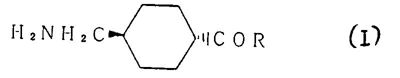

## Description

### [TECHNICAL FIELD]

This invention relates to a tranexamic acid derivatives and an external preparation for skin including the same and more particularly, to an improvement of depigmentation effect thereof.

### [BACKGROUND ART]

While the mechanisms of pigmentation such as melasma, chloasma, and the like in skin are not completely understood, it is believed that a melanin pigment is formed and abnormally deposited in the skin due to hormone abnormalities and UV light irradiation caused by sunlight. The above-mentioned pigmentation is generally treated by administrating substances suppressing the formation of melanin. For example, vitamin C is administrated in a large amount; glutathione or a similar compound is injected; or L-ascorbic acid, kojic acid, cysteine, or a similar compound is applied to localized areas in the form of an ointment, cream or lotion.

On the other hand, in the United States and Europe, hydroquinone preparations are used as pharmaceuticals.

However, the depigmentation effects of the above mentioned compounds except for hydroquinone are very week.

### [DISCLOSURE OF THE INVENTION]

Accordingly, it is an object of the present invention to eliminate the above described problems of the prior art and to provide a compound which has an excellent depigmentation effect, high safety and stability and an external preparation for skin including the same.

As a result of studies undertaken by the present inventors so as to attain this aim, it has been found that new types of tranexamic acid derivatives have excellent depigmentation effect and skin care effect comparing with the hydroquinone. The present invention has been achieved on the basis of this findings.

Namely, in the first aspect of the present invention, there are provided tranexamic acid derivatives or the salts thereof represented by the following general formula 1. 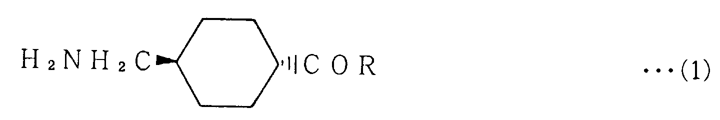
Wherein R in the above formula 1 represents a residue including a six membered ring bound by an ester bonding or an amide bonding.

In the second aspect of the present invention, there are provided tranexamic acid derivatives or the salts thereof of which R is a gentisic acid residue as represented by the structural formula 2. 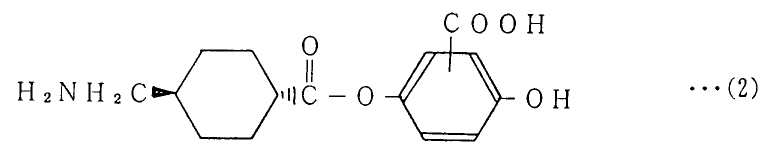
In the third aspect of the present invention, there are provided tranexamic acid derivatives or the salts thereof of which R is a tranexamic acid residue as represented by the following structural formula 3. 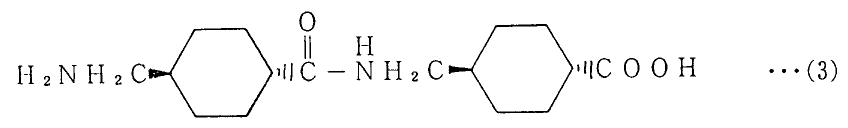
In the fourth aspect of the present invention, there are provided tranexamic acid derivatives or the salts thereof of which R is a hydroquinone residue as represented by the following structural formula 4. 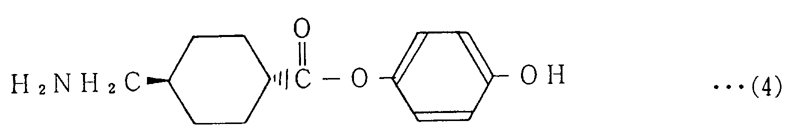
In the fifth aspect of the present invention, there is provided an external preparation for skin comprising the tranexamic acid derivatives or the salts thereof represented by the above formulas 1 to 4.

The compositions of the present invention are explained in detail herein after.

The tranexamic acid derivatives according to the present invention are classified into the ester of tranexamic acid and gentisic acid and the salt thereof, the dimer of tranexamic acid and the salt thereof, and the ester of tranexamic acid and hydroquinone and the salt thereof.

The esters of tranexamic acid-gentisic acid and the salts thereof are new compounds and it is possible to synthesize them according to the following method.

The ester of tranexamic acid-gentisic acid is produced by reacting gentisic acid to tranexamic acid or a reactive derivative thereof. For examples of the reactive derivatives of tranexamic acid, acid halide such as acid chloride and acid bromide, and mixed acid anhydride are cited. In regards to reaction of tranexamic acid, it is preferable to react them in a solvent which does not influence the reaction. For examples of the solvent, an organic solvents such as dichloromethane, dichloroethane, chloroform, acetonitrile and benzene are cited. The reaction is preferably progressed for 1∼8 hours in a range from a room temperature to a boiling temperature of the solvent. In case of reacting the tranexamic acid itself, it is preferable to use a condensing agent such as dicyclohexyl carbodiimide. An amino group of the tranexamic acid may be protected by a protective group such as a benzyloxy carbonyl group. The protective group is removed by a catalytic reduction after esterification. A carboxyl group and/or other an phenolic hydroxyl group of the gentisic acid may be protected by an appropriate protective group such as a benzyl group. The protective group is removed by a catalytic reduction after esterification.

The ester of tranexamic acid-gentisic acid produced by the above method may be in form of a salt of inorganic acid such as hydrochloric acid, sulfuric acid, phosphoric acid and hydrobromic acid; a salt of organic acid such as acetic acid, lactic acid, maleic acid, fumaric acid, tartaric acid, citric acid, methane sulfonic acid and p-toluenesulfonic acid; a sodium salt, a potassium salt, an ammonium salt, a magnesium salt or a calcium salt.

For examples of the compound, 2-(trans-4-aminomethyl cyclohexyl carbonyloxy)-5-hydroxy benzoic acid, 5-(trans-4-aminomethyl cyclohexyl carbonyloxy)-2-hydroxy benzoic acid, 2-(trans-4-aminomethyl cyclohexyl carbonyloxy)-5-hydroxy benzoic acid hydrochloride, sodium 2-(trans-4-aminomethyl cyclohexyl carbonyloxy)-5-hydroxy benzoate, 5-(trans-4-aminomethyl cyclohexyl carbonyloxy)-2-hydroxy benzoic acid hydrochloride, potassium 5-(trans-4-aminomethyl cyclohexyl carbonyloxy)-2-hydroxy benzoic acid are cited.

The dimer of tranexamic acid is expressed as trans-4-(trans-4-aminomethyl cyclohexane carbonyl) aminomethyl cyclohexane carboxylic acid.

The dimer of tranexamic acid and the salt thereof are new compounds and it is possible to synthesize them according to the following method.

An reactive derivative of tranexamic acid or protected tranexamic acid of which an amino group is protected by an appropriate protective group such as a benzyloxy carbonyl group is produced. On the other hand, protected tranexamic acid of which a carbonyl group is protected by a protective group such as a benzyl group is produced. The dimer of tranexamic acid is produced by reacting the tranexamic acid or a reactve derivative of which the amino group is protected and the tranexamic acid or the protected tranexamic acid of which the carboxyl group is protected. In case of using the protected tranexamic acid, the protective group is removed by catalytic reduction after dimerization. For examples of the reactive derivatives, acid halide such as acid chloride and acid bromide; mixed acid anhydride and active ester are preferably cited. In regards to reaction of the protected tranexamic acid, it is preferable to react them in a solvent which does not influence the reaction. For examples of the solvent, organic solvents such as dichloromethane, dichloroethane, chloroform, acetonitrile and benzene are cited. The reaction is preferably progressed for 1 to 24 hours in a range from room temperature to boiling temperature of the solvent. In case of reacting the tranexamic acid itself, it is preferable to use condensing agents such as dicyclohexyl carbodiimide.

The dimer of tranexamic acid which is produced by the above method may be in form of a salt of inorganic acid such as hydrochloric acid, sulfuric acid, phosphoric acid and hydrobromic acid; a salt of organic acid such as acetic acid, lactic acid, maleic acid, fumaric acid, tartaric acid, citric acid and methane sulfonic acid; an inorganic salt such as a sodium salt, a potassium salt, an ammonium salt, an magnesium salt and a calcium salt; or an organic salt such as monoethanol amine, diethanol amine and triethanol amine.

The ester of tranexamic acid-hydroquinone and the salt thereof according to the present invention is expressed as 4'-hydroxyphenyl trans-4-amino methyl cyclohexane carboxylate.

The ester of tranexamic acid-hydroquinone and the salt thereof according to the present invention are new compounds and it is possible to synthesize them according to the following method.

Reactive derivatives of tranexamic acid or protected tranexamic acid of which an amino group is protected by a protective group such as a benzyloxy carbonyl group are produced. A reactive derivative is reacted with hydroquinone or protected hydroquinone of which a phenolic hydroxyl group is protected by a protective group such as a benzyl group and the ester of tranexamic acid-hydroquinone is produced. In case of using the protected tranexamic acid and/or protected hydroquinone, the protective groups are removed by catalytic reduction after producing the ester. The examples of reactive derivatives, acid halide such as acid chloride and acid bromide, and mixed acid anhydride are preferably cited. In regards to reaction of tranexamic acid, it is preferable to react them in a solvent which does not influence the reaction. For examples of the solvent, dichloromethane, dichloro ethane, chloroform, acetonitrile and benzene are cited. The reaction is preferably progressed for 1 to 8 hours in a range from room temperature to boiling temperature of the solvent.

The compounds of the invention which are produced by the above method may be in form of salt of inorganic acid such as hydrochloric acid, sulfuric acid, phosphoric acid and hydrobromic acid; or a salt of organic acid such as acetic acid, lactic acid, maleic acid, fumaric acid, tartaric acid, citric acid, methane sulfonic acid and p-toluen sulfonic acid.

An external preparation for skin according to the present invention comprises at least one of the tranexamic derivatives and the content of this ingredient is in a range from 0.001 to 20 weight % and more preferably from 0.01 to 7 weight % to the total amount of external preparation for skin.

In case that the content of tranexamic acid derivatives is less than 0. 001wt%, the depigmentation effect and skin care effect might not be enough, and even if the amount of tranexamic acid derivatives are more than 20 weight %, the further improvement of these effect might not be obtained.

It is possible to add other components which are usually used for cosmetics or medicine to the external preparation for skin according to the present invention. For examples of the components, oily ingredients, ultraviolet absorbents, antioxidants, surfactants, moisturing ingredients, perfume, water, alcohol, thickener, dye, nutritive ingredients for skin (tocopheryl acetate, pantothenyl ethyl ether, salt of glycyrrhizic acid) are cited.

### [BEST MODE FOR CARRYING OUT THE INVENTION]

The present invention will be explained in detail with reference to following examples. It is to be understood, however, that the present invention is not restricted thereto.

### EXAMPLE 1 ESTER OF TRANEXAMIC ACID - GENTISIC ACID

Synthesis examples of the ester of tranexamic acid-gentisic acid and the salt thereof according to the present invention will be explained in detail.

### SYNTHESIS EXAMPLE 1

The ester of gentisic acid and tranexamic acid is obtained by producing the protected tranexamic acid and protected gentisic acid, producing a protected ester and removing the protective group from the protected bodies. The detailed examples are explained herein after.

### ① Tranexamic acid protected by a benzyloxy carbonyl protective group (Trans-4-benzyloxy carbonyl aminomethyl cyclohexane carboxylic acid)

Benzyloxy carbonyl chloride (8.2g, 48mmol) and 10% sodium hydroxide aqueous solution (20ml) were gradually dropped into 10% sodium hydroxide aqueous solution of tranexamic acid (6.3g, 40mmol) under stirring and cooling condition. After stirring the reaction mixture for one hour, crystal was obtained by adding hydrochloric acid to the above reaction mixture and filtering. Recrystallization was conducted by using benzene-petroleum ether. As a result, the tranexamic acid protected by benzyl hydroxy carbonyl protective group (10.7g, yield: 92%) was obtained.
Melting point 114∼116°C

### ② Gentisic acid protected by a dibenzyl protective group (Benzyl 5-benzyloxy-2-hydroxy benzoate)

The gentisic acid (10g, 65mmol) was reacted with benzyl bromide (20ml, 130mmol) for 10 hours in acetone including potassium carbonate in room temperature. The reaction mixture was separated and purified by a silica gel column. As a result, the gentisic acid protected by a dibenzyl protective group (14.0g, yield 83%) was obtained.
Melting point 68.5∼69.0°C

### ③ Protected ester of tranexamic acid-gentisic acid (Benzyl 5-benzyloxy-2-(trans-4-benzyloxy carbonyl aminomethyl cyclohexyl carbonyloxy) benzoate)

Trans-4-benzyloxy carbonyl amino methyl cyclohexane carboxylic acid (4.4g, 15mmol) was added to thionyl chloride (50ml). Petroleum ester (50ml) was added to the reaction mixture after reacting for 30 minutes at 40°C. As a result, white colored crystal was obtained by filtering. Dried benzene solution (50ml) of the white crystal was gradually dropped to dried benzene solution in which benzyl 5-benzyloxy-2-hydroxy benzoate (3. 75g, 14.5mmol) and triethyl amine (1.63g, 16mmol) were dissolved and stirred for 4 hours. Triethyl amine hydrochloride which precipitated in the solution were filtered off. The reaction mixture was separated and purified by a silica gel column and the protected ester of tranexamic acid-gentisic acid (4.02g yield 52%) was obtained.
Melting point: 118.5∼119°C

### ④ Ester of tranexamic acid-gentisic acid (2-(trans-4-aminomethyl cyclohexyl carbonyloxy)-5-hydroxy benzoic acid)

Benzyl 5-benzyloxy-2-(trans-4-benzyloxy carbonyl aminomethyl cyclohexyl carbonyloxy) benzoate (1.07g, 2mmol) was dissolved in acetic acid (100ml), 10% palladium carbon (100mg) was added and catalytic reduction was carried out in room temperature under atomospheric pressure. After theoretical amount of hydrogen was absorbed, the catalyst was filtered off. The solution was evaporated by the reduced pressure. A residue was crystallized by using ether and the ester of tranexamic acid-gentisic acid was obtained as a white colored crystal (438mg, yield: 100%).
Melting point: 196∼198.5°C

### ⑤ The ester of tranexamic acid-gentisic acid hydrochloride (2-(4-aminomethyl cyclohexyl carbonyloxy)-5-hydroxy benzoic acid hydrochloride)

2-(4-aminomethyl cyclohexyl carbonyloxy)-5-hydroxy benzoic acid (500mg) was treated with 9% hydrochloric acid acetic acid solution-ether and recrystallized from ethanol ether. As a result, the ester of tranexamic acid-gentisic acid hydrochloride was obtained as a white colored crystal (420mg, yield 72%).
Melting point: 246.5∼ 248.5°C
¹H -NMR(DMSO-d₆, TMS, ppm)
δ 1.01-2.51(m, 10H, cyclohexane ring)
δ 2.66(d,2H J=6.8Hz, -CH₂NH₂)
δ 6.91(d,1h, J=8.5Hz, benzene ring H-3)
δ 6.98(dd,1H,J=2.9 and 8.5 Hz, benzene ring H-4)
δ 7.28(d,1H, J=2.9 Hz, benzene ring H-6)
δ 8.05(bs, 3H, -NH₃⁺Cl⁻)
δ 9.75(bs, 1H, -OH)
δ 12.74(bs, 1H, -COOH)
¹³C-NMR(DMSO-d₆, TMS, ppm)
δ 27. 6(cyclohexane ring C-3, C-5)
δ 28.6(cyclohexane ring C-2,C-6)
δ 34.9(cyclohexane ring C-4)
δ 42.0(cyclohexane ring C-1)
δ 44.1(-CH₂NH₂)
δ 117.0(benzene ring C-6)
δ 120.0(benzene ring C-4)
δ 124.4(benzene ring C-3)
δ 124.6(benzene ring C-1)
δ 142.0(benzene ring C-2)
δ 154.8(benzene ring C-5)
δ 165.6(ester C=0)
δ 173. 7(-COOH)

| Elementary analysis | | | | |
|---|---|---|---|---|
| Calculated for C₁₅H₁₉NO₅·HCl | C:54.63, | H:6.11, | N:4.25, | Cl:10.75% |
| Found | C:54.62, | H:6.12, | N:4.22, | Cl:10.72% |

### SYNTHESIS EXAMPLE 2

Benzyl 2,5-dihydroxy benzoate was produced according to the synthesis example 1, ②.
Melting point: 101.5 ∼ 103.0°C
Benzyl 5-(trans-4-benzyloxy carbonyl aminomethyl cyclohexyl carbonyloxy)-2-hydroxy benzoate was produced according to the synthesis example 1, ③.
Melting point: 110 ∼111°C
5-(4-aminomethyl cyclohexyl carbonyloxy)-2-hydroxy benzoic acid hydrochloride was produced according to the synthesis example 1 ④, ⑤.
Melting point: 181∼185°C
¹H-NMR(DMSO-d₆, TMS, ppm)
δ 1.02∼2.55 (m,10H, cyclohexane ring)
δ 2.68(t, 2H, J=5.9Hz, -CH₂NH₂)
δ 6.98(d, 1H, J=8.8Hz,benzene ring H-3)
δ 7.25(dd,1H, J=2.9 and 8.8 Hz, benzene ring H-4)
δ 7.47(d,1H, J=2.9HZ, benzene ring H-6)
δ 8.11(bs,3H,-NH₃⁺Cl)

| Elementary analysis | | | | |
|---|---|---|---|---|
| Calculated for C₁₅H₁₉NO₅·HCl | C:54.63, | H:6.11, | N:4.25, | Cl:10.75% |
| Found | C:54.64, | H:6.08, | N:4.22, | Cl:10.78% |

The effect of ester of tranexamic acid-gentisic acid according to the example 1 is examined. The result is shown herein after.

### (1) EXAMINATION FOR DEPIGMENTATION EFFECT

60 subjects were disposed under sunlight for 4 hours in summer (2 hours × 2 days). The lotions were applied to skin of medial brachia for once in each morning and evening from 5 days after the day when the skin was exposed to the sun light for eight weeks. The one group consists of 10 subjects and six groups were given for the examination in accordance with the following prescriptions.

### SAMPLES FOR EXAMPLE 1-1∼1-3 and COMPARISON 1∼3

| ( Alcohol phase) | weight% |
|---|---|
| 95% Ethyl alcohol | 25.0 |
| Polyoxyethylene (25 mols) hydrogenated caster oil ether | 2.0 |
| Antioxidant, antiseptic | q.s. |
| Perfume | q.s. |
| compound (described in the TABLE-1) | 1.0 |

| (Aqueous phase) | |
|---|---|
| Glycerin | 5.0 |
| Sodium hexametaphosphate | q.s. |
| Ion exchanged water | Balance |

### (Production method)

The aqueous phase and the alcohol phase were prepared respectively, and both phase were mixed and dissolved.

### (Evaluation method)

Depigmentation effect after the application of the samples were evaluated according to the following standard.

### (Standard)

Ⓞ: The number of subjects who judged the result was "extremely effective" and "effective" were not less than 80%.
○: The number of subjects who judged the result was "extremely effective" and "effective" were from 50% to 80%.
△: The number of subjects who judged the result was "extremely effective" and "effective" were from 30% to 50%.
X: The number of subjects who judged the result was "extremely effective" and "effective" were less than 30 %.

**TABLE 1**

| | COMPOUND | DEPIGMENTATION EFFECT |
|---|---|---|
| COMP.1 | none | X |
| COMP.2 | hydroquinone | △ |
| COMP.3 | tranexamic acid | ○ |
| EXM.1-1 | 2-(trans-4-aminomethyl cyclohexyl carbonyloxy 5-hydroxy benzoic acid | Ⓞ |
| EXM.1-2 | 5-(trans-4-aminomethyl cyclohexyl carbonyloxy 2-hydroxy-benzoic acid | Ⓞ |
| EXM.1-3 | 2-(trans-4-aminomethyl cyclohexyl hydroxy benzoic acid hydrochloride | Ⓞ |

As is clear from the TABLE-1, the tranexamic acid derivatives according to the example 1-1,1-2 and 1-3 are excellent in the effect to prevent the deposition of melanin pigmentation after the skin was exposed to the sunlight comparing with the hydroquinone or tranexamic acid, and it was recognized that the examples prevented the skin from suntan.

### (2) EXAMINATION FOR SKIN CARE EFFECT

### EXAMINATION METHOD

The cosmetics according to the examples 1-1, 1-2 and 1-3 were applied to left halves of face of subject, and the cosmetic according to the comparison 1 was applied to right halves of the face of subjects after washing the face every morning and night for two weeks. One group of subjects consists of ten women and 3 groups of the subjects were given for the examination.

### (Evaluation method)

The effectiveness of three items (moisturising effect, texture of skin surface, maintenance (one day) of moisturising effect) were evaluated according to the following standards.

### (Standard)

Ⓞ: The number of subjects who judged the result was "extremely effective" and "effective" were not less than 80 %.
○: The number of subjects who judged the result was "extremely effective" and "effective" were from 50% to 80%.
△: The number of subjects who judged the result was "extremely effective" and "effective" were from 30% to 50%.
X: The number of subjects who judged the result was "extremely effective " and "effective" were less than 30%.

**TABLE 2**

| RESULTS | | | |
|---|---|---|---|
| | MOISTURISING EFFECT | TEXTURE OF SKIN SURFACE | MAINTENANCE OF MOISTURISING EFFECT |
| COMP.1. | X | X | X |
| EXM. 1-1 | Ⓞ | Ⓞ | Ⓞ |
| EXM. 1-2 | Ⓞ | Ⓞ | Ⓞ |
| EXM. 1-3 | Ⓞ | Ⓞ | Ⓞ |

As is clear from the TABLE 2, the examples had excellent skin care effect such as moisturising effect, texture of skin surface, and maintenance of moisturising effect comparing with the comparison 1.

Examples of external preparation for skin comprising the ester of tranexamic acid-gentisic acid according to the example 1 are explained herein under. Any of external preparation for skin had a excellent depigmentation effect and skin care effect.

### EXAMPLE 1-1 CREAM

| | weight% |
|---|---|
| Stearic acid | 5.0 |
| Stearyl alcohol | 4.0 |
| Isopropyl myristate | 18.0 |
| Glycerin monostearate | 3.0 |
| Propylene glycol | 10.0 |
| 2-(trans-4-aminomethyl cyclohexyl carbonyloxy)-5-hydroxy benzoic acid hydrochloride | 20.0 |
| Potassium hydroxide | 0.2 |
| Sodium bisulfite | 0.01 |
| Antiseptic | q.s. |
| Perfume | q.s. |
| Ion exchanged water | balance |

### (Production method)

The propylene glycol and the potassium hydroxide were added to the ion exchanged water and kept at 70°C (Aqueous phase). The other ingredients are mixed and dissolved at 70°C (Oil phase). The oil phase was gradually added to the aqueous phase and kept the temperature for a while so as to complete reaction. The mixture was uniformly emulsified with a Homomixer and cooled down to 30°C under stirring condition.

### EXAMPLE 1-2 CREAM

| | weight% |
|---|---|
| Stearic acid | 6.0 |
| Sorbitan monostearate ester | 2.0 |
| Polyoxyethylene(20 moles)sorbitan monostearate | 1.5 |
| Propylene glycol | 10.0 |
| 5-(trans-4-aminomethylcyclohexyl carbonyloxy)-2-hydroxy benzoic acid hydrochloride | 7.0 |
| Glycerin trioctanoate | 10.0 |
| Squalane | 5.0 |
| Sodium bisulfite | 0.01 |
| Ethyl p-hydroxy benzoate | 0.3 |
| Perfume | q.s. |
| Ion exchanged water | balance |

### (Production method)

The propylene glycol was added to the ion exchanged water and kept at 70°C (Aqueous phase). The other ingredients were mixed and dissolved at 70°C (Oil phase). The oil phase was added to the aqueous phase gradually and preemulsified, then uniformly emulsified with a Homo mixer and cooled down to 30°C under stirring condition.

### EXAMPLE 1-3 CREAM

| | weight% |
|---|---|
| Stearyl Alcohol | 7.0 |
| Stearic acid | 2.0 |
| Hydrogenated lanolin | 2.0 |
| Squalane | 5.0 |
| 2-octyldodecyl alcohol | 6.0 |
| Polyoxy ethylene(25 moles)cetyl alcohol ether | 3.0 |
| Glycerin monostearate | 2.0 |
| Propylene glycol | 5.0 |
| 2-(trans-4-aminomethyl cyclohexyl carbonyloxy)-5-hydroxy benzoic acid | 0.005 |
| Perfume | q.s. |
| Sodium bisulfite | 0.03 |
| Ethyl P-hydroxybenzoate | 0.3 |
| Ion exchanged water | balance |

### (Production method)

The propylene glycol was added to the ion exchanged water and kept at 70°C (Aqueous phase). The other ingredients were mixed and dissolved at 70°C (Oil phase). The oil phase was gradually added to the aqueous phase and preemulsified, and then uniformly emulsified with a Homomixer and cooled down to 30°C under stirring condition.

### EXAMPLE 1-4 MILKY LOTION

| | weight% |
|---|---|
| Stearic acid | 2.5 |
| Cetyl alcohol | 1.5 |
| Vaseline | 5.0 |
| Liquid paraffin | 10.0 |
| Polyoxyethylene (10 moles) monooleate | 2.0 |
| Polyethylene glycol 1500 | 3.0 |
| Triethanol amine | 1.0 |
| 5-(trans-4-aminomethyl cyclohexyl carbonyloxy) 2-hydroxy benzoic acid | 10.0 |
| Sodium bisulfite | 0.01 |
| Ethyl P-hydroxybenzoate | 0.3 |
| Carboxy vinyl polymer | 0.05 |
| Perfume | q.s. |
| Ion exchanged water | balance |

### (Production method)

The carboxy vinyl polymer was uniformly dissolved in small amount of the ion exchanged water (A phase). The polyethylene glycol 1500 and the triethanol amine were added to the other part of ion exchanged water and dissolved at 70°C (water phase). The other ingredients were dissolved under heating condition at 70°C (Oil phase). The oil phase was added to the aqueous phase and preemulsified . The A phase was added to the mixture and emulsified uniformly with a Homomixer and cooled down to 30°C under stirring condition.

### EXAMPLE 1-5 MILKY LOTION

| (Oil phase) | weight% |
|---|---|
| Stearyl alcohol | 1.5 |
| Squalane | 2.0 |
| Vaseline | 2.5 |
| Deodorized liquid lanolin | 1.5 |
| Evening primrose oil | 2.0 |
| Isopropyl myristate | 5.0 |
| Glycerin monooleate | 2.0 |
| Polyoxyethylene (60 moles) hydrogenated caster oil | 2.0 |
| Tocopherol acetate | 0.05 |
| Ethyl p-hydroxybenzoate | 0.2 |
| Butyl p-hydroxybenzoate | 0.1 |
| 5-(trans-4-aminomethyl cyclohexyl carbonyloxy) -2-hydroxy benzoic acid | 1.0 |
| 2-(trans-4-aminomethyl cyclohexyl carbonyloxy) -5-hydroxy benzoic acid | 1.0 |
| Perfume | q.s. |

| (Aqueous phase) | |
|---|---|
| Sodium bisulfite | 0.01 |
| Glycerin | 5.0 |
| Sodium hyaluronate | 0.01 |
| Carboxy vinyl polymer | 0.2 |
| Potassium hydroxide | 0.2 |
| Ion exchanged water | balance |

### (Production method)

The oil phase was dissolved at 70°C. The aqueous phase was dissolved at 70 °C. The Oil phase was added in the aqueous phase and emulsified by a emulsifier and cooled down to 30°C by a heat exchanger.

### EXAMPLE 1-6 JELLY

| | weight% |
|---|---|
| 95% Ethyl alcohol | 10.0 |
| Dipropylene glycol | 15.0 |
| Polyoxyethylene (50 moles) oleyl alcohol ether | 2.0 |
| Carboxy vinyl polymer | 1.0 |
| Sodium hydroxide | 0.15 |
| L-arginine | 0.1 |
| 5-(trans-4-aminomethyl cyclohexyl carbonyloxy)-2-hydroxy benzoic acid hydrochloride | 1.0 |
| 2-(trans-4-aminomethyl cyclohexyl carbonyloxy)-5-hydroxy benzoic acid hydrochloride | 1.0 |
| Methyl p-hydroxy benzoate | 0.2 |
| Perfume | q.s. |
| Ion exchanged water | balance |

### (Production method)

The carboxyvinyl polymer was uniformly dissolved in the ion exchanged water. The 5-(trans-4-aminomethyl cyclohexyl carbonyloxy)-2-hydroxy benzoic acid hydrochloride, the 2-(trans-4-aminomethyl cyclohexyl carbonyloxy)-5-hydroxy benzoic acid hydrochloride and the polyoxyethylene (50moles) oleyl alcohol ether were dissolved in the 95% ethanol and the mixture was added into the aqueous phase. The other ingredients were added to the mixture and the sodium hydroxide and the L-arginine were added for neutralizing and thickening.

### EXAMPLE 1-7 SKIN LOTION

| (A phase) | weight% |
|---|---|
| Ethanol (95%) | 10.0 |
| Polyoxyethylene(20moles)octyldodecanol | 1.0 |
| Methyl-p-hydroxy benzoate | 0.15 |
| Pantothenyl ethyl ether | 0.1 |
| 2-(trans-4-aminomethyl cyclohexyl carbonyloxy)-5-hydroxy benzoic acid | 0.05 |

| (B phase) | |
|---|---|
| Potassium hydroxide | 0.1 |

| (C phase) | |
|---|---|
| Glycerin | 5.0 |
| Dipropylene glycol | 10.0 |
| Sodium bisulfite | 0.03 |
| Carboxyvinyl polymer | 0.2 |
| Purified water | balance |

### (Production method)

The A phase and C phase were uniformly dissolved, respectively. The C phase was added to the A phase and dissolved. The B phase was added to the mixture and packed.

### EXAMPLE 1-8 PACK

| (A phase) | weight% |
|---|---|
| Dipropylene glycol | 5.0 |
| Polyoxyethylene (60 moles)hydrogenated caster oil | 5.0 |

| (B phase) | |
|---|---|
| 2-(trans-4-aminomethyl cyclohexyl carbonyloxy)-5-hydroxy benzoic acid hydrochloride | 1.0 |
| 5-(trans-4-aminomethyl cyclohexyl carbonyloxy)-2-hydroxy benzoic acid hydrochloride | 1.0 |
| Olive oil | 5.0 |
| Tocopherol acetate | 0.2 |
| Ethyl p-hydroxy benzoate | 0.2 |
| Perfume | 0.2 |

| (C phase) | |
|---|---|
| Sodium bisulfite | 0.03 |
| Polyvinyl alcohol(Saponification degree: 90, polymerization degree: 2000) | 13.0 |
| Ethanol | 7.0 |
| Purified water | balance |

### (Production method)

The A phase, B phase and C phase were uniformly dissolved respectively. The B phase was added to the A phase and dissolved. The C phase was added to the mixture and packed.

### EXAMPLE 2 DIMER OF TRANEXAMIC ACID

The synthesis example of the dimer of tranexamic acid and the salt thereof according to the present invention is explained herein after.

### SYNTHESIS EXAMPLE

### ① Tranexamic acid protected by benzyloxy carbonyl protective group (Trans-4-benzyloxy carbonyl aminomethyl cyclohexane carboxylic acid).

Benzyloxy carbonyl chloride (8.2g, 48mmol) and 10% sodium hydroxide aqueous solution (20ml) were gradually dropped into 10% sodium hydroxide aqueous solution of tranexamic acid (6.3g, 40mmol) under cooling and stirring condition. After stirring for one hour under cooling condition, hydrochloric acid was added to the reaction mixture so as to adjust the reaction mixture to acid. The crystal was obtained by filtering. The tranexamic acid protected by benzyloxy carbonyl group (10.7g, yield: 92%) was obtained by recrystallization from benzene-petroleum ether.
Melting point: 154∼155°C

### ② Tranexamic acid protected by a benzyl protective group (Benzyl trans-4-aminomethyl cyclohexane carboxylate hydrochloride)

Tranexamic acid (10.0g, 63mmol) was added to thionyl chloride (40ml), and ethyl ether (60ml) was added in the reaction mixture after stirring it for 1 hour in room temperature. Precipitated crystal was obtained by filtering from the reaction mixture. The crystal was added to dried dioxane solution of benzyl alcohol [benzyl alcohol (7.5ml, 70mmol) was dissolved in the dried dioxane] and the system was reacted for one hour at 80°C. Precipitated crystal was obtained by filtrating after cooling. The crystal was recrystallized from ethanol and as a result, the tranexamic acid protected by the benzyl protective group (13.8g, yield: 76%) was obtained.
Melting point : 154∼155°C

### ③ Protected dimer of tranexamic acid (Benzyl trans-4-(trans-4-benzyloxy carbonyl aminomethyl cyclohexane carbonyl) aminomethyl cyclohexane carboxylate)

Trans-4-benzyloxy carbonyl aminomethyl cyclohexane carboxylic acid (5.00g, 17mmol) was added to thionyl chloride (5ml) and petroleum ether (50ml) was added in the system after reacting for 30 min. at 40°C. Precipitated white colored crystal was obtained by filtering. Dried benzene solution (50ml) of the white colored crystal was gradually dropped in the dried benzene solution (200ml) of benzyl trans-4-aminomethyl cyclohexane carboxylate hydrochloride (4.87g, 17mmol) and triethyl amine (3.30 g, 37mmol). The reaction mixture was further stirred for 20 hours. White colored crystal precipitated in the reaction mixture was obtained by filtering, and separated and purified by a silica gel column. As a result, the protected dimer of tranexamic acid (3.28g, yield 36%) was obtained.
Melting point: 174 ∼ 175°C

### ④ Dimer of tranexamic acid (Trans-4-(trans-4-aminomethyl cyclohexane carbonyl) aminomethyl cyclohexane carboxylic acid hydrochloride)

Benzyl trans-4(trans-4-benzyloxy carbonyl aminomethyl cyclohexane carbonyl) aminomethyl cyclohexane carboxylate (5.12g, 9.83mmol) was dissolved in acetic acid (150mmol), 10% palladium carbon (600mg) was added to the reaction mixture and catalytic reduction was carried out in room temperature under atomospheric pressure.

After theoretical amount of hydrogen was absorbed, catalyst was removed by filtering, the solution was evaporated by reduced pressure and residue was crystallized from the 10% hydrochloric acid acetic acid solution-ether. The obtained crystal was recrystallized from ethanol-ether and the dimer of tranexamic acid (2.82g, yield: 86%) was obtained as a salt of hydrochloric acid.
Melting point : 290°C(decomposition)
¹H-NMR(DMSO-d₆ ,TMS, ppm)
δ 0.82∼2.15(m,20H,cyclohexane ring)
δ 2.62( d,2H,J=6.8Hz,-CH₂NH₃⁺CL⁻)
δ 2.88( t,2H,J=6.2 Hz,-CH₂NH⁻)
δ 7.55( bs, 1H,-CH₂NH⁻)
δ 8.03( bs,3H,NH₃+Cl⁻)
δ 11.74(bs,1H,-COOH)
¹³C-NMR(DMSO-d₆, TMS, ppm)
δ 28.2(cyclohexane ring)
δ 28.4(cyclohexane ring)
δ 29.0(cyclohexane ring)
δ 29.3(cyclohexane ring)
δ 34.9(cyclohexane ring)
δ 36.9(cyclohexane ring)
δ 42.4(cyclohexane ring)
δ 43.5(cyclohexane ring)
δ 44.2, δ 44.3(- CH₂NH₃⁺Cl⁻ and -CH₂NH⁻)
δ 174.7 (amid C=0)
δ 176.4(-COOH)

| Elmentary analysis | | | | |
|---|---|---|---|---|
| Calculated for C₁₆H₂₈N₂O₃·HCl | C:57.73, | H:8.78, | N:8.42, | Cl:10.65% |
| Found | C:57.62, | H:8.85, | N:8.22, | Cl:10.81% |

The effect of external preparations for skin including the dimer of tranexamic acid was examined.

### (1) EXAMINATION FOR DEPIGMENTATION EFFECT

### Examination method

Depigmentation effect was examined by using the following external preparations for skin in accordance with the above described condition. Result is shown in the TABLE-3.

### SAMPLE FOR EXAMPLE 2-1,2-2, AND COMPARISON 1,2,3

| (Alcohol phase) | weight% |
|---|---|
| 95% ethyl alcohol | 25.0 |
| Polyethylene (25 moles)hydrogenated caster ether | 2.0 |
| Antioxidant, Antiseptic | q.s. |
| Perfume | q.s. |
| Compounds (described in the TABLE-3) | 1.0 |

| (Aqueous phase) | |
|---|---|
| Glycerin | 5.0 |
| Sodium hexametaphosphate | q.s. |
| Ion exchanged water | balance |

### (Production method)

The alcohol phase and aqueous phase were prepared respectively, and both phase were mixed and dissolved.

**TABLE 3**

| | COMPOUNDS | DEPIGMENTATION EFFECT |
|---|---|---|
| COMP.1 | none | X |
| COMP.2 | Hydroquinone | △ |
| COMP.3 | Tranexamic acid | ○ |
| EXM.2-1 | Trans-4-(trans-4-aminomethyl cyclohexane carbonyl) aminomethyl cyclohexane carboxylic acid | Ⓞ |
| EXM.2-2 | Trans-4-(trans-4-aminomethyl cyclohexyl carbonyl) aminomethyl cyclohexane carboxylic acid hydrochloride | Ⓞ |

As is shown in the TABLE-3, the effect of the examples after exposed to the sunlight was superior to the effect of comparisons in view of inhibition of the deposition of melanin pigment and prevention of suntan.

### (2) Skin care effect

Skin care effect of the examples 2-1 and 2-2 was examined in accordance with the above-mentioned condition. Result is shown in the TABLE-4.

**TABLE 4**

| RESULT | | | |
|---|---|---|---|
| | MOISTURISING EFFECT | TEXTURE OF SKIN SURFACE | MAINTENANCE OF MOISTURISING EFFECT |
| COMP.1 | X | X | X |
| EXM.2-1 | Ⓞ | Ⓞ | Ⓞ |
| EXM.2-2 | Ⓞ | Ⓞ | Ⓞ |

As is clear from the TABLE-4, the examples had excellent skin care effect such as moisturising effect, texture of skin surface, and maintenance of moisturising effect comparing with the comparison 1.

Examples of external preparation for skin according to the example 1 are explained herein under. Any of external preparation for skin had a excellent depigmentation effect and skin care effect.

### EXAMPLE 2-1 CREAM

| | weight% |
|---|---|
| Stearic acid | 5.0 |
| Stearyl alcohol | 4.0 |
| Isopropyl myristate | 18.0 |
| Glycerin monostearate ester | 3.0 |
| Propylene glycol | 10.0 |
| Trans-4-(trans-4-aminomethyl cyclohexane carbonyl) aminomethyl cyclohexane carboxylic acid hydrochloride | 20.0 |
| Potassium hydroxide | 0.01 |
| Sodium bisulfite | q.s. |
| Antiseptic | q.s. |
| Ion exchanged water | balance |

### (Production method)

The propylene glycol and the potassium hydroxide were added to the ion exchanged water and kept at 70°C (Aqueous phase). The other ingredients were mixed and dissolved at 70 °C (Oil phase). The oil phase was gradually added to the aqueous phase and kept the temperature for a while after end of the adding so as to complete reaction. The mixture was uniformly emulsified with a Homomixer and cooled down to 30 °C under stirring condition.

### EXAMPLE 2-2 CREAM

| | weight% |
|---|---|
| Stearic acid | 6.0 |
| Sorbitan monostearate | 2.0 |
| Polyoxyethylene(20 moles)sorbitan monostearate | 1.5 |
| Propylene glycol | 10.0 |
| Trans-4-(trans-4-aminomethyl cyclohexane carbonyl) aminomethyl cyclohexane carboxylic acid | 7.0 |
| Glycerin trioctanoate | 10.0 |
| Squalane | 5.0 |
| Sodium bisulfite | 0.01 |
| Ethyl p-hydroxy benzoate | 0.3 |
| Perfume | q.s. |
| Ion exchanged water | balance |

### (Production method)

The propylene glycol was added to the ion exchanged water and kept at 70°C (Aqueous phase). The other ingredients were mixed and dissolved at 70°C (Oil phase). The oil phase was gradually added to the aqueous phase and preemulsified. The mixture was uniformly emulsified with a Homomixer and cooled down to 30°C under stirring condition.

### EXAMPLE 2-3 CREAM

| | weight% |
|---|---|
| Stearyl alcohol | 7.0 |
| Stearic acid | 2.0 |
| Hydrogenated lanolin | 2.0 |
| Squalane | 5.0 |
| 2-octyl dodecyl alcohol | 6.0 |
| Polyoxyethylene(25 moles)cetyl alcohol ether | 3.0 |
| Glycerin monostearate | 2.0 |
| Propylene glycol | 5.0 |
| Trans-4-(trans-4-aminomethyl cyclohexane carbonyl)aminomethyl cyclohexane carboxylic acid hydrochloride | 0.005 |
| Perfume | q.s. |
| Sodium bisulfite | 0.03 |
| Ethyl p-hydroxybenzoate | 0.3 |
| Ion exchanged water | balance |

### (Production method)

The propylene glycol was added to the ion exchanged water and kept at 70°C (Aqueous phase). The other ingredients were mixed and dissolved at 70 °C (Oil phase). The oil phase was gradually added to the aqueous phase and preemulsified. The mixture was then uniformly emulsified with a Homomixer and cooled down to 30°C under stirring condition.

### EXAMPLE 2-4 MILKY LOTION

| | weight% |
|---|---|
| Stearic acid | 2.5 |
| Cetyl alcohol | 1.5 |
| Vaseline | 5.0 |
| Liquid paraffin | 10.0 |
| Polyoxyethylene(10 moles)monooleate | 2.0 |
| Polyethylene glycol 1500 | 3.0 |
| Triethanol amine | 1.0 |
| Trans-4-(trans-4-amino methyl cyclohexane carbonyl) aminomethyl cyclohexane carboxylic acid hydrochloride | 10.0 |
| Sodium bisulfite | 0.01 |
| Ethyl p-hydroxybenzoate | 0.3 |
| Carboxy vinyl polymer | 0.05 |
| Perfume | q.s. |
| Ion exchanged water | balance |

### (Production method)

The carboxy vinylpolymer was dissolved in a part of the ion exchanged water (A phase). The polyethylene glycol 1500 and the triethanolamine were added to the other part of ion exchanged water and dissolved at 70°C (Aqueous phase). The other ingredients were dissolved under heating condition at 70°C (Oil phase). The A phase was added to the mixture and emulsified uniformly with a Homomixer and cooled down to 30°c under stirring condition.

### EXAMPLE 2-5 MILKY LOTION

| (Oil phase) | weight% |
|---|---|
| Stearyl alcohol | 1.5 |
| Squalane | 2.0 |
| Vaseline | 2.5 |
| Deodorized liquid lanolin | 1.5 |
| Evening primrose oil | 2.0 |
| Isopropyl myristate | 5.0 |
| Glycerin monooleate | 2.0 |
| Polyoxyethylene (60 moles)hydrogenated caster oil | 2.0 |
| Tocopherol acetate | 0.05 |
| Ethyl p-hydroxy benzoate | 0.2 |
| Butyl p-hydroxy benzoate | 0.1 |
| Trans-4-(trans-4-aminomethyl cyclohexane carbonyl)aminomethyl cyclohexane carboxylic acid | 1.0 |
| Trans-4-(trans-4-aminomethyl cyclohexane carbonyl)aminomethyl cyclohexane carboxylic acid hydrochloride | 1.0 |
| Perfume | q.s. |

| (Aqueous phase) | |
|---|---|
| Sodium bisulfite | 0.01 |
| Glycerin | 5.0 |
| Sodium hyaluronate | 0.01 |
| Carboxy vinyl polymer | 0.2 |
| Potassium hydroxide | 0.2 |
| Purified water | balance |

### (Production method)

The oil phase was dissolved at 70°C. The aqueous phase was dissolved at 70°C. The Oil phase was mixed into the aqueous phase and emulsified by an emulsifier and cooled down to 30°C by a heat exchanger.

### EXAMPLE 2-6 JELLY

| | weight% |
|---|---|
| 95% Ethyl alcohol | 10.0 |
| Dipropylene glycol | 15.0 |
| Polyoxyethylene (50 moles)oleyl alcohol ether | 2.0 |
| Carboxy vinyl polymer | 1.0 |
| Sodium hydroxide | 0.15 |
| L-arginine | 0.1 |
| Trans-4-(trans- 4-aminomethyl cyclohexane carbonyl) aminomethyl cyclohexane carboxylic acid | 2.0 |
| Methyl p-hydroxy benzoate | 0.2 |
| Perfume | q.s. |
| Ion exchanged water | balance |

### (Production method)

The carboxy vinyl polymer was uniformly dissolved in the ion exchanged water. The trans-4-(trans-4-aminomethyl cyclohexane carbonyl) aminomethyl cyclohexane carboxylic acid and the polyoxyethylene (50 moles) oleyl alcohol ether were dissolved in the 95% ethanol and the mixture was added into the aqueous phase. The other ingredients were added to the mixture and, the sodium hydroxide and the L-Arginine were added for neutralizing and thickening.

### EXAMPLE 2-7 SKIN LOTION

| (A phase) | weight% |
|---|---|
| Ethanol (95%) | 10.0 |
| Polyoxyethylene(20 moles)octyldodecanol | 1.0 |
| Methyl p-hydroxybenzoate | 0.15 |
| Pantothenyl ethyl ether | 0.1 |
| Trans-4-(trans-4-aminomethyl cyclohexane carbonyl) aminomethyl cyclohexane carboxylic acid | 0.05 |

| (B phase) | |
|---|---|
| Potassium hydroxide | 0.1 |

| (C phase) | |
|---|---|
| Glycerin | 5.0 |
| Dipropylene glycol | 10.0 |
| Sodium bisulfite | 0.03 |
| Carboxy vinyl polymer | 0.2 |
| Purified water | balance |

### (Production method)

The A phase and C phase were uniformly dissolved respectively. The A phase was added to the C phase and dissolved. The B phase was added to the mixture and packed.

### EXAMPLE 2-8 PACK

| (A phase) | weight% |
|---|---|
| Dipropylene glycol | 5.0 |
| Polyoxyethylene(60 moles)hydrogenated caster oil | 5.0 |

| (B phase) | |
|---|---|
| Trans-4-(trans-4-aminomethyl cyclohexane carbonyl)aminomethyl cyclohexane carboxylic acid hydrochloride | 1.0 |
| Olive oil | 5.0 |
| Tocopherol acetate | 0.2 |
| Ethyl p-hydroxybenzoate | 0.2 |
| Perfume | 0.2 |

| (C phase) | |
|---|---|
| Sodium bisulfite | 0.03 |
| Polyvinyl alcohol(Saponification degree 90, Polymerization degree 2,000) | 13.0 |
| Ethanol | 7.0 |
| Purified water | balance |

### (Production method)

The A phase, B phase and C phase were uniformly dissolved respectively. The B phase was added to the A phase and dissolved. The C phase was added to the mixture and packed.

### EXAMPLE 3 ESTER OF TRANEXAMIC ACID - HYDROQUINONE

The synthesis example of ester of tranexamic acid-hydroquinone and the salt thereof according to the present invention is explained herein after.

### ① Tranexamic acid protected by a benzyloxy carbonyl chloride group (Trans-4-benzyloxy carbonyl aminomethyl cyclohexane carboxylic acid)

Benzyloxy carboxylic chloride (8.2g, 48mmol) and 10% sodium hydroxide solution (20ml) were dropped in 10% sodium hydroxide solution (16ml) of tranexamic acid (6.3g, 40mmol) under stirring and cooling condition. After stirring for one hour under cooling condition, hydrochloric acid was added into the reaction mixture so as to adjust the reaction mixture to acid and crystal was obtained by filtering. The crystal was recrystallized from benzene-petroleum ether and then the tranexamic acid protected by benzyloxy carbonyl protective group (10.7g, yield: 92%) was obtained.
Melting point : 114∼116°C

### ② Protected ester of tranexamic acid-hydroquinone (4'-benzyloxyphenyl trans-4-benzyloxy carbonyl aminomethyl cyclohexane carboxylate)

Trans-4-benzyloxy carbonyl aminomethyl cyclohexane carboxylic acid (4.4g, 15mmol) was added into thionyl chloride (5ml). After reacted for 30 minutes at 40°C, petroleum ether (50ml) was added into the reaction mixture. As a result, white colored crystal was obtained by filtering. The dried benzene solution (50ml) of the white crystal was gradually dropped to dried benzene solution (50ml) of hydroquinone monobenzyl ether (Made by Wako junyaku) (4.0g, 17.5mmol) and triethyl amine (2.02g, 20mmol) under stirring in room temperature and further stirred for 4 hours. Triethyl amine hydrochloride which was precipitated in the mixture was removed by filtering, the reaction mixture was separated and purified by a silica gel column. As a result, the protected ester of tranexamic acid-hydroquinone (4.32g, yield: 57%) was obtained.
Melting point : 131∼ 132°C

### ③ Ester of tranexamic acid-hydroquinone hydrochloride (4'-hydroxy phenyl trans-4-aminomethyl cyclohexane carboxylate hydrochloride)

4'-benzyloxy phenyl trans-4-benzyloxy carbonyl aminomethyl cyclohexane carboxylate (3.9g, 7.7mmol) was dissolved in acetic acid (150ml), 10% palladium carbon (400mg) was added and catalytic reduction was carried out in room temperature under atomospheric pressure. After the theoretical amount of hydrogen was absorbed, the catalyst was removed by filtering, the solution was evaporated by the reduced pressure. The residue was crystallized by 9% hydrochloric acid acetic acid solution. As a result, the ester of tranexamic acid- hydroquinone hydrochloride (2.2g, yield 100%) was obtained.
Melting point : 232.5∼ 233°C
¹H-NMR(DMSO-d₆, TMS, ppm)
δ 1.00-2.50(m.10H cyclohexane ring)
δ 2.65(d,2H,J=6.8Hz,-CH₂NH₃⁺Cl⁻)
δ 6.77(d,2H,J=9.3Hz,benzene ring)
δ 6.87(d,2H,J=8.8Hz,benzene ring)
δ 8.12(bs,3H, NH₃⁺Cl⁻)
δ 9.53(bs,1H, -OH)

| Elementary analysis | | | | |
|---|---|---|---|---|
| Calculated for C₁₄H₁₇NO₃·HCl | C:58.84, | H:7.05, | N:4.90, | Cl:12.41% |
| Found | C:58.62, | H:7.12, | N:4.72, | Cl:12.57% |

### (1) EXAMINATION FOR DEPIGMENTATION EFFECT

Depigmentation effect was examined by using the following external preparations for skin in accordance with the above described condition. Result is shown in the TABLE-5.

### SAMPLE FOR EXAMPLE 3-1,3-2, AND COMPARISON 1,2,3

| (Alcohol phase) | weight% |
|---|---|
| 95% ethyl alcohol | 25.0 |
| Polyethylene(25 moles)hydrogenated caster ether | 2.0 |
| Antioxidant, Antiseptic | q.s. |
| Perfume | q.s. |
| Compounds(described in the TABLE-5) | 1.0 |

| (Aqueous phase) | |
|---|---|
| Glycerin | 5.0 |
| Sodium hexametaphosphate | q.s. |
| Ion exchanged water | balance |

### (Production method)

The alcohol phase and aqueous phase were prepared respectively, and both phase were mixed and dissolved.

**TABLE 5**

| | CHEMICAL COMPOUNDS | DEPIGMENTATION EFFECT |
|---|---|---|
| COMP.1 | none | X |
| COMP.2 | Hydroquinone | △ |
| COMP.3 | Tranexamic acid | △ |
| EXM.3-1 | 4'-hydroxy phenyl trans-4-aminomethyl cyclohexane carboxylate | Ⓞ |
| EXM.3-2 | 4'-hydroxy phenyl trans-4-aminomethyl cyclohexane carboxylate hydrochloride | Ⓞ |

As is shown in the TABLE-5, the effect of the examples after exposed to the sunlight were superior to the effect of comparisons in view of inhibition of the deposition of melanin pigment and prevention of suntan.

### (2) SKIN CARE EFFECT

Skin care effect of the examples was examined in accordance with the above-mentioned condition. Result is shown in the TABLE-6.

**TABLE 6**

| EFFECT | | | |
|---|---|---|---|
| | MOISTURISING EFFECT | TEXTURE OF SKIN SURFACE | MAINTENANCE OF MOISTURISING EFFECT |
| COMP.1 | X | X | X |
| EXM.3-1 | Ⓞ | Ⓞ | Ⓞ |
| EXM.3-2 | Ⓞ | Ⓞ | Ⓞ |

As is clear from the TABLE-6, the examples had excellent skin care effect such as moisturising effect, texture of skin surface, and maintenance of moisturising effect comparing with the comparison 1.

Examples of external preparation for skin according to the example 3 comprising the ester of tranexamic acid-hydroquinone are explained herein under. Any of external preparation for skin had excellent depigmentation effect and skin care effect.

### EXAMPLE 3-1 CREAM

| | weight% |
|---|---|
| Stearic acid | 5.0 |
| Stearyl alcohol | 4.0 |
| Isopropyl myristate | 18.0 |
| Glycerin monostearate ester | 3.0 |
| Propylene glycol | 10.0 |
| 4-hydroxy phenyl trans-4-aminomethyl cyclohexane carboxylate hydrochloride | 20.0 |
| Potassium hydroxide | 0.2 |
| Sodium bisulfite | 0.01 |
| Antiseptic | q.s. |
| Perfume | q.s. |
| Ion exchanged water | balance |

### (Production method)

The propylene glycol and the potassium hydroxide were added to the ion exchanged water and kept at 70°C (Aqueous phase). The other ingredient are mixed and dissolved at 70°C (Oil phase). The oil phase was gradually added to the aqueous phase and kept at some temperature for a while after the adding for reaction so as to complete reaction. The mixture was uniformly emulsified with a Homomixer and cooled down to 30°c under stirring condition.

### EXAMPLE 3-2 CREAM

| | weight% |
|---|---|
| Stearic acid | 6.0 |
| Sorbitan monostearate | 2.0 |
| Polyoxy ethylene(20 moles)sorbitan monostearate ester | 1.5 |
| Propylene glycol | 10.0 |
| 4'-hydroxy phenyl trans-4-aminomethyl cyclohexane carboxylate | 7.0 |
| Glycerin trioctanoate | 10.0 |
| Squalane | 5.0 |
| Sodium bisulfite | 0.01 |
| Ethyl p-hydroxybenzoate | 0.3 |
| Perfume | q.s. |
| Ion exchanged water | balance |

### (Production method)

The propylene glycol was added to the ion exchanged water and kept at 70° C (Aqueous phase). The other ingredients were mixed and dissolved at 70°C (Oil phase). The oil phase was gradually added to the aqueous phase and preemulsified, and then uniformly emulsified with a Homomixer and cooled down to 30°C under stirring condition.

### EXAMPLE 3-3 CREAM

| | weight% |
|---|---|
| Stearyl alcohol | 7.0 |
| Stearic acid | 2.0 |
| Hydrogenated lanolin | 2.0 |
| Squalane | 5.0 |
| 2-octyldodecyl alcohol | 6.0 |
| Polyoxyethylene(25 moles)cetyl alcohol ether | 3.0 |
| Glycerin monostearate ester | 2.0 |
| Propylene glycol | 5.0 |
| 4'-hydroxy phenyl trans-4-aminomethyl cyclohexane carboxylate ester | 0.005 |
| Perfume | q.s. |
| Sodium bisulfite | 0.03 |
| Ethyl p-hydroxy benzoate | 0.3 |
| Ion exchanged water | balance |

### (Production method)

The propylene glycol was added to the ion exchanged water and kept at 70°C (Aqueous phase). The other ingredients were mixed and dissolved at 70°C (Oil phase). The oil phase was gradually added to the aqueous phase and preemulsified. The mixture was uniformly emulsified with a Homomixer and cooled down to 30°C under stirring condition.

### EXAMPLE 3-4 MILKY LOTION

| | weight% |
|---|---|
| Stearic acid | 2.5 |
| Cetyl alcohol | 1.5 |
| Vaseline | 5.0 |
| Liquid paraffin | 10.0 |
| Polyoxyethylene(10 moles)monooleate | 2.0 |
| Polyethylene glycol 1500 | 3.0 |
| Triethanolamine | 1.0 |
| 4'-hydroxy phenyl trans-4-aminomethyl cyclohexane carboxylate hydrochloride | 10.0 |
| Sodium bisulfite | 0.01 |
| Ethyl p-hydroxybenzoate | 0.3 |
| Carboxy vinyl polymer | 0.05 |
| Perfume | q.s. |
| Ion exchanged water | balance |

### (Production method)

The carboxy vinyl polymer was dissolved in a part of the ion exchanged water (A phase). The polyethylene glycol 1500 and the triethanolamine were added to the other part of the ion exchanged water and dissolved at 70°C (aqueous phase). The other ingredients were dissolved under heating condition at 70°C (Oil phase). The oil phase was added to the aqueous phase and preemulsified. The A phase was added to the mixture and emulsified uniformly with a Homomixer and cooled down to 30°C under stirring condition.

### EXAMPLE 3-5 MILKY LOTION

| (Oil phase) | weight% |
|---|---|
| Stearyl alcohol | 1.5 |
| Squalane | 2.0 |
| Vaseline | 2.5 |
| Deodorized liquid lanolin | 1.5 |
| Evening primrose oil | 2.0 |
| Isopropyl myristate | 5.0 |
| Glycerin monooleate | 2.0 |
| Polyoxyethylene(60 moles)hydrogenated caster oil | 2.0 |
| Tocopherol acetate | 0.05 |
| Ethyl p-hydroxybenzoate | 0.2 |
| Butyl p-hydroxybenzoate | 0.1 |
| 4'-hydroxy phenyl trans-4-aminomethyl cyclohexane carboxylate hydrochloride | 1.0 |
| 4'-hydroxy phenyl trans-4-aminomethyl cyclohexane carboxylate | 1.0 |
| Perfume | q.s. |

| (Aqueous phase) | |
|---|---|
| Sodium bisulfite | 0.01 |
| Glycerin | 5.0 |
| Sodium hyaluronate | 0.01 |
| Carboxy vinyl polymer | 0.2 |
| Potassium hydroxide | 0.2 |
| Purified water | balance |

### (Production method)

The oil phase was dissolved at 70°c. The aqueous phase was dissolved at 70°C. The oil phase was mixed in the aqueous phase and emulsified by an emulsifier and cooled down to 30°C by a heat exchanger.

### EXAMPLE 3-6 JELLY

| | weight% |
|---|---|
| 95% Ethyl alcohol | 10.0 |
| Dipropylene glycol | 15.0 |
| Polyoxyethylene (50 moles) oleyl alcohol ether | 2.0 |
| Carboxy vinyl polymer | 1.0 |
| Sodium hydroxide | 0.15 |
| L-arginine | 0.1 |
| 4'-hydroxy phenyl trans-4-aminomethyl cyclohexane carboxylate hydrochloride | 2.0 |
| Methyl p-hydroxybenzoate | 0.2 |
| Perfume | q.s. |
| Ion exchanged water | balance |

### (Production method)

The carboxy vinyl polymer was uniformly dissolved in the ion exchanged water. The 4'-hydroxy phenyl trans-4-aminomethyl cyclohexane carboxylate hydrochloride and the polyoxyethylene(50 moles)oleyl alcohol ether were dissolved in the 95% ethanol and the mixture was added into the aqueous phase. The other ingredients were added to the mixture and the sodium hydroxide and the L-arginine were added for neutralizing and thickening.

### EXAMPLE 3-7 SKIN LOTION

| (A phase) | weight% |
|---|---|
| Ethanol (95%) | 10.0 |
| Polyoxy ethylene(20 moles) octyldodecanol | 1.0 |
| Methyl p-hydroxy benzoate | 0.15 |
| Pantothenyl ethyl ether | 0.1 |
| 4'-hydroxy phenyl trans-4-aminomethyl cyclohexane carboxylate | 0.005 |

| (B phase) | |
|---|---|
| Potassium hydroxide | 0.1 |

| (C phase) | |
|---|---|
| Glycerin | 5.0 |
| Dipropylene glycol | 10.0 |
| Sodium bisulfite | 0.23 |
| Carboxy vinyl polymer | 0.2 |
| Purified water | balance |

### (Production method)

The A phase and the C phase were uniformly dissolved, respectively. The A phase was added to the C phase and dissolved. The B phase was added to the mixture and packed.

### EXAMPLE 3-8 PACK

| (A phase) | weight% |
|---|---|
| Dipropylene glycol | 5.0 |
| Polyoxyethylene (60 moles) hydrogenated caster oil | 5.0 |

| (B phase) | |
|---|---|
| 4'-hydroxy phenyl trans-4-aminomethyl cyclohexane carboxylate hydrochloride | 1.0 |
| Olive oil | 5.0 |
| Tocopherol acetate | 0.2 |
| Ethyl p-hydroxy benzoate | 0.2 |
| Perfume | 0.2 |

| (C phase) | |
|---|---|
| Sodium bisulfite | 0.03 |
| Polyvinyl alcohol (Saponification degree 90, polymerization degree 2,000) | 13.0 |
| Ethanol | 7.0 |
| Purified water | balance |

### (Production method)

The A phase, B phase and C phase were uniformly dissolved respectively. The B phase was added to the A phase and dissolved. The C phase was added to the mixture and packed.

The tranexamic acid derivatives according to the present invention are new compounds which have excellent depigmentation effect and skin care effect. Furthermore, the external preparations for skin comprising the tranexamic acid derivatives are new external preparation for skin which have depigmentation effect and skin care effect.

## Claims

1. Tranexamic acid derivatives or the salts thereof represented by the following general formula(1); 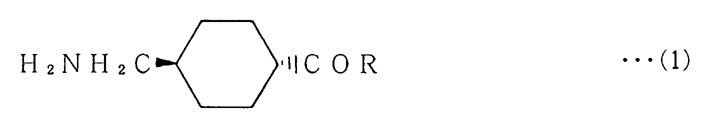 wherein R in the above formula 1 represents a residue including a six membered ring bound by an ester bonding or an amide bonding.

2. The tranexamic acid derivatives or the salts thereof according to claim 1; wherein said R represents a gentisic acid residue as represented by the following structural formula 2;

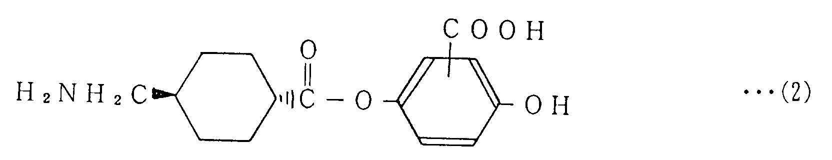

3. The tranexamic acid derivatives or the salts thereof according to claim 1, wherein said R represents a tranexamic acid residue as represented by the following structural formula 3.

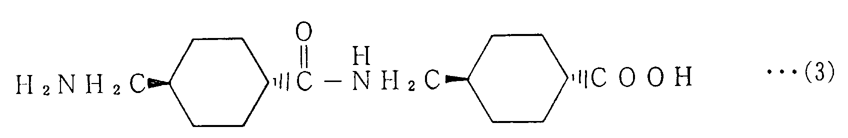

4. The tranexamic derivatives or the salts thereof according to claim 1, wherein said R represents a hydroquinone residue as represented by the following structural formula 4.

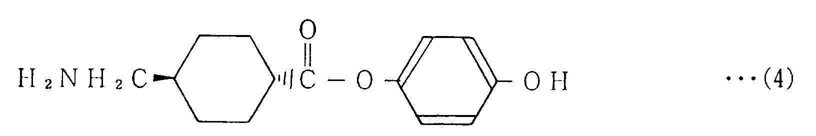

5. An external preparation for skin comprising at least one of the tranexamic acid derivatives according to the claims 1 to 4.
